Europäisches Patentamt

European Patent Office

Office européen des brevets

(11) Veröffentlichungsnummer: **0 054 627**
**B1**

(12) **EUROPÄISCHE PATENTSCHRIFT**

(45) Veröffentlichungstag der Patentschrift:
20.03.85

(51) Int. Cl.⁴: **C 11 B 11/00,** A 61 K 7/48,
A 61 K 35/78

(21) Anmeldenummer: **81107592.8**

(22) Anmeldetag: **23.09.81**

(54) Arzneimittel und Kosmetikum zur äusseren Anwendung.

(30) Priorität: **22.12.80 DE 3048507**

(43) Veröffentlichungstag der Anmeldung:
**30.06.82 Patentblatt 82/26**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**GB - A - 630 271**

**CHEMICAL ABSTRACTS, Band 35, 1941, Spalte 16569
COLUMBUS OHIO (US) & Ann. Chim. Applicata 30, 303-7
(1940) L. MONTI "A wax extracted from hemp"**

(73) Patentinhaber: **Füssener Textil AG,
D-8958 Füssen/Ostallgäu (DE)**

(72) Erfinder: **Stüber, Siegfried, Dr., Drehergasse 46,
D-8958 Füssen/Ostallgäu (DE)**

(74) Vertreter: **Berg, Wilhelm, Dr. et al, Patentanwälte Dr.
Berg Dipl.-Ing. Stapf Dipl.-Ing. Schwabe Dr. Dr. Sandmair
Postfach 86 02 45 Stuntzstrasse 16,
D-8000 München 86 (DE)**

## Beschreibung

Flachs- und Hanfwachse sind bekannte Produkte (Technologie der Textilfasern, Der Flachs, Band V, 1.1.1930,129–141 und 283; Hofmeister Ber., 1903, 1047, Ullmanns Enzyklopädie der technischen Chemie, 18. Band, 1967, 287; Technische Fortschrittsberichte, Band XXXI, Wachse, wachsähnliche Stoffe und technische Wachsgemenge, 1934, 41, 47; Z. Angew. Chem., Band I, 1919, 126). Die beiden Wachse sind sowohl in chemischer als auch in physikalischer Hinsicht zum Teil übereinstimmend, zum Teil sehr ähnlich.

Zur Herstellung geht man im allgemeinen von feinteiligen flachs- oder hanfwachshaltigen Abfällen aus, wie sie beispielsweise bei der Aufbereitung der Flachs- und Hanfstengel zur Fasergewinnung, insbesondere beim Knicken und Schwingen und der Grünentholzung, aber auch in den weiteren Stufen bis einschliesslich dem Verspinnen, anfallen. Der Flachswachsgehalt dieser mehr oder weniger feinteiligen Abfälle kann, von den groben, Scheben und kurze Wergteile enthaltenden Flachsstäuben ansteigend, bis über 20 Gew.% bei feinen und feinsten Stäuben betragen. Es ist bekannt, dass sich die Wachse aus diesen Abfällen mit Alkoholen, Benzinen, Benzol, Aceton, Äther und Mischungen derselben extrahieren und aus dem Extrakt durch Abziehen der Lösungsmittel gewinnen lassen.

Für die Gewinnung der Wachse in technischem Umfang ist ein solches Vorgehen wegen der schlechten Ausbeuten unbefriedigend. An Versuchen, eine Erhöhung der Ausbeute zu erzielen, hat es nicht gefehlt. So ist es bekannt, Flachsabfälle, insbesondere Flachsstäube, zunächst in Rührwerken mit einer mehrfachen Wassermenge zwischen 30°C und Dampftemperaturen zu behandeln. Die Massen werden dann abfiltriert, die von wasserlöslichen Stoffen befreiten, ausgelaugten Rückstände zunächst getrocknet und schliesslich die so erhaltenen, von Wasser befreiten Flachsabfälle mit Kohlenwasserstoffen, Benzinen oder Alkoholen extrahiert. Durch Einengen des Extrakts und/oder durch Ausfällung daraus konnten bis zu 22 Gew.% Rohwachse, bezogen auf das Ausgangsmaterial, erhalten werden (DE-C 817 335). Weitere Verfahren zur Gewinnung von Hanf- bzw. Flachswachs z.B. durch Extraktion sind in der GB-A 630 721 und in Chemical Abstracts 1941, vol. 35, 1656/9 beschrieben.

Es wurde nun gefunden, dass Hanfwachs bestimmte bioaktive Eigenschaften hat. Gegenstand der Erfindung sind danach ein Arzneimittel und Kosmetikum zur äusseren Anwendung, das aus Hanfwachs besteht oder Hanfwachs enthält.

Mit Vorteil werden hierbei solche Hanfwachse verwendet, die aus kleinteiligen, hanfwachshaltigen Abfällen unter Extraktion mittels organischer Lösungsmittel und Isolierung aus dem Extrakt, wobei die Extraktion mit aliphatischen Halogenkohlenwasserstoffen erfolgt, gewonnen sind. Überraschenderweise hat sich gezeigt, dass bei Verwendung aliphatischer Halogenkohlenwasserstoffe, insbesondere aliphatischer Chlorkohlenwasserstoffe zur Extraktion der Hanfwachse aus solchen enthaltenden Abfällen das Gewinnungsverfahren nicht nur wesentlich vereinfacht werden kann, sondern die Ausbeute sogar noch erhöht werden kann, insbesondere auch gegenüber den weit umständlicheren und aufwendigeren vorbekannten Verfahren. So bedarf es dabei keineswegs der bislang notwendigen Behandlung mit einer mehrfachen Wassermenge bei Temperaturen zwischen 30°C und Dampftemperaturen. Weiter kann dann die bislang zwingend erforderliche Trocknungsstufe der wasserbehandelten, wachshaltigen Stäube entfallen, was, abgesehen von den apparativen Vorteilen, auch eine beachtliche Energieeinsparung ermöglicht. Diese beachtlichen Vorteile werden dabei nicht etwa mit einer Verringerung der Ausbeute erkauft, sondern diese Verfahrensweise führt bei hanfwachshaltigen Abfällen zu Wachsausbeuten bis in den Bereich von 10 bis 15%.

Bei der Durchführung des Verfahrens haben sich insbesondere solche Lösungsmittel der vorgenannten Art bewährt, deren Siedepunkte zwischen 60–100°C, insbesondere zwischen 65 oder 70–10°C liegen.

Die diskontinuierliche Extraktion kann in der Wärme unter Rühren durchgeführt werden, jedoch wird die Extraktion in der Wärme, insbesondere unter Rückflussbedingungen, bevorzugt. So kann die Extraktion in technischem Umfang beispielsweise mit bekannten Vorrichtungen, wie kontinuierlich arbeitenden Extraktoren unter Rückfluss, durchgeführt werden. Besonders bewährt haben sich Lösungsmittel vom Typ des halogenierten, insbesondere des chlorierten Äthans, namentlich mit Siedepunkten im Bereich von ca. 70–100°C, wie beispielsweise 1,1,1-Trichloräthan oder 1,2-Dichloräthan. Als sehr gut brauchbar haben sich aber auch Lösungsmittel vom Typ des halogenierten, insbesondere chlorierten Äthylens erwiesen, wie beispielsweise 1,1,2-Trichloräthylen. Die wachshaltigen Lösungen können von den unlöslichen Rückständen z.B. durch Filtration usw. gewonnen werden.

Die Gewinnung der Wachse aus ihren Lösungen in aliphatischen Halogen-Kohlenwasserstoffen kann z.B. durch Abdestillieren der Lösungsmittel, gegebenenfalls unter reduziertem Druck, durch Ausfällen der Wachse aus ihren Lösungen, z.B. durch Abkühlen oder durch Verdrängen, durch Zugabe von die Wachse nicht lösenden Stoffen, usw. erfolgen. Die Lösungsmittel können dabei, gegebenenfalls unter Zwischenreinigung, im Kreislauf geführt, wieder verwendet werden. Häufig hat es sich als Vorteil erwiesen, die nach Entfernen der aliphatischen Halogenkohlenwasserstoffe erhaltenen Wachse in halogenfreien Lösungsmitteln, wie Alkoholen, Ketonen usw. nochmals zu lösen und zur Entfernung etwaiger geringer Gehalte an halogenierten Lösungsmitteln einer Destillation unter Entfernung der Lösungsmittel zu unterwerfen.

In technischem Umfang wird es zur Gewinnung der Wachse aus ihren Lösungen bevorzugt, das Lösungsmittel unter vermindertem Druck abzudestillieren, die Wachse in heissem Äthanol zu lösen und das Äthanol unter reduziertem Druck abzudestillieren. Dies wird gegegebenfalls öfter wiederholt, damit die Wachse frei vom chlorierten Lösungsmittel werden. Die von den Lösungsmittel befreiten Wachse können in üblicher Weise weiterverarbeitet werden, beispielsweise raffiniert, gebleicht, über Aktivkohle abfiltriert usw. werden.

Die erfindungsgemässen Hanfwachs-Arzneimittel und kosmetischen Mittel besitzen, abgesehen von den hier nicht betroffenen Wachsen aus indischem Hanf, wertvolle pharmakologische und kosmetische Eigenschaften. Dies gilt insbesondere bei Erkrankungen des Bewegungsapparates, Prellungen, Schwellungen, Muskelverspannungen, als auch bei Erkrankungen des rheumatischen Formenkreises, Ischias, Gicht, Arthriden, Arthrose und auch für die Hautbehandlung, wie Entzündungen, Insektenstiche, Sonnenbrand und erschlaffte Haut.

Die Erfindung wird nachfolgend anhand von Beispielen erläutert.

Darstellung von Hanfwachs

1 kg feinster Hanfstaub wird mit 4 Liter 1,1,1-Trichloräthan bis zur Erschöpfung (etwa 10 Stunden lang) kontinuierlich extrahiert. Der grösste Teil des im Staub gebundenen Lösungsmittels (ca. 80%) wird dem Nasstaub durch Abnutschen entzogen. Der noch restliche Teil des im Staub gebundenen Lösungsmittels (ca. 15%) wird durch Einbringen von 1 Liter Wasser (T = 60°C) und weiteres Abnutschen, wobei die organische Phase von der Wasserphase abgetrennt wird, entfernt. Somit lassen sich nach der Extraktion und der Verdrängung mit warmem Wasser über 95% des Lösungsmittels wieder erneut einsetzen. Die Verdrängung mit Warmwasser wird umso vollständiger, je mehr Wasser verwendet wird. Die vereinigte Hanfwachslösung wird am Rotationsverdampfer vom Lösungsmittel befreit, wobei die Wasserbadtemperatur zum Schluss auf etwa 80°C gesteigert wird. Das zurückbleibende Wachs wird noch zweimal mit Äthanol gelöst und das Äthanol wiederum abgezogen. Dadurch wird ein, auch für therapeutische Zwecke geeignetes, von 1,1,1-Trichloräthan freies Hanfwachs erhalten. Das Produkt kann mehrfach umkristallisiert und schliesslich mit Aktivkohle gereinigt werden. Die Ausbeute an Hanfwachs betrug 126 g bezogen auf 1 kg Feinstaub, entsprechend einer Ausbeute von 12,6%.

Es wurden Ausbeuten zwischen 10 und 15% je nach Staubfeinheit gefunden.

| | |
|---|---|
| Schmelzpunkt | 69°C |
| Verseifungszahl | 101 |
| Jodzahl | 22,5 |
| Säurezahl | 13,6 |
| spez. Gewicht | 0,98 |
| Unverseifbares | 11,0% |

Verwendung von Hanfwachs

Das Hanfwachs kann mit bekannten Excipienten, Hilfsstoffen, Trägern, Konservierungsmittel und dergl. in üblicher Weise konfektioniert werden. Dabei ist es beispielsweise zur Bereitung von Bädern durchaus möglich, Hanfwachs enthaltenden Staub unmittelbar dem Bad zuzusetzen oder in einen Filterbeutel einzubringen. Hanfwachs hat sich als Salbengrundlage wegen seiner Wirkstoffe bewährt. Daher lassen sich sowohl Heilsalben mit den schon beschriebenen Wirkungen herstellen als auch kosmetische Salben, wobei auch hier die in Hanfwachs vorhandenen Wirkstoffe wesentlich zur bioaktiven Wirkung beitragen. Dies wird durch die nachfolgenden Beispiele erläutert.

1. Salbe

| | |
|---|---|
| Hanfwachs Natur, grün | 18% |
| Glycerin | 5% |
| Neutralöl (Triglyceride) | 72% |
| Äthanol | 3% |
| Kollagen | 1,5% |
| Zitronensaft | 0,5% |

Die Salbe hat sich besonders bewährt bei Erkrankungen des rheumatischen Formenkreises, wie Ischias, Gicht, Arthriden, Athrose, Muskelverspannungen, Erkrankungen des Bewegungsapparates, Bandscheibenbeschwerden, Prellungen, Entzündungen (u.a. Sehnenscheidenentzündung) und dergl. Zudem ist die Salbe durch in ihr enthaltene natürlich konservierende Hanfflavyliumfarbstoffe vor bakteriellem und mycotischem Abbau geschützt. Die Farbe der Salbe variiert je nach eingesetztem Hanfwachs. Farbe und Geruch des verwendeten Hanfwachses können beispielsweise durch Vakuumbehandlung in Gegenwart geruchsbindender Stoffe oder durch Umkristallisation in Gegenwart von Absorptionsmitteln erfolgen.

2. Salbe

| | |
|---|---|
| Hanfwachs farblos | 12% |
| Glycerin | 6% |
| Neutralöl (Triglyceride) | 65% |
| Kollagen | 5% |
| Äthanol | 3,5% |
| Zitronensaft | 0,5% |
| Wasser | 8% |

Diese Salbe hat sich, wie schon beschrieben, besonders bewährt bei Entzündungen, Insektenstichen, Sonnenbrand und erschlaffter Haut (Gesichtsmaske).

3. Creme

Zur Herstellung von Feuchtigkeitscremes auf nicht fettender Grundlage kann Hanfwachs auch in Gelee eingearbeitet werden.

4. Rheumabad

Hanfwachshaltiger Staub wird nach Einstellung eines schwachsauren pHs mittels eines Acetatpuffers in einen Filterbeutel verpackt, der beim Einbringen in das warme Wasser sofort Wirkstoffe abgibt.

5. Packungen

Hanfwachshaltiger Staub wird mit einem Acetatpuffer schwach-sauer eingestellt und die Masse in einen Beutel eingebracht. Nach Anfeuchten mit Wasser und Erhitzen sind Packungen erhältlich, die insbesondere bei Bandscheibenbeschwerden und Entzündungen gute Wirkung gezeigt haben.

**Patentanspruch**

1. Arzneimittel und Kosmetikum zur äusseren Anwendung, dadurch gekennzeichnet, dass es aus Hanfwachs besteht oder Hanfwachs enthält.

**Claim**

1. Medicament and cosmetic for external application characterised in that it consists of or contains "hemp wax".

**Revendication**

1. Médicament et produit cosmétique pour usage externe, caractérisé par le fait qu'il est constitué de cire de chanvre ou qu'il contient de la cire de chanvre.